# EUROPÄISCHE PATENTANMELDUNG

(11) **EP 1 949 934 A1**
(43) Veröffentlichungstag der Anmeldung: **30.07.2008**
(21) Anmeldenummer: 08101043.1
(22) Anmeldetag: 29.01.2008
(51) Int. Cl.: A61N 1/36, A61N 1/05

(54) **Vorrichtung und Verfahren zur Kontrolle einer Körperfunktion**

(30) Priorität: 29.01.2007 DE 102007005212
(71) Anmelder: Biobedded Systems GmbH, 46045 Oberhausen (DE)
(72) Erfinder: Hilburg, Andreas, 46045 Oberhausen (DE)
(74) Vertreter: Patentanwälte Freischem

(57) **Zusammenfassung**

Die Erfindung betrifft eine Vorrichtung (1) und ein Verfahren zur Kontrolle einer Körperfunktion, beispielsweise eine Muskeispannung, der Herzrhythmus oder dergleichen. Der Erfindung liegt die Aufgabe zugrunde, eine Vorrichtung (1) und ein Verfahren zur Kontrolle einer Körperfunktion, insbesondere zur Durchführung einer Inkontinenz-Therapie zu schaffen, die zu jedem Zeitpunkt diskret verwendbar sind. Erfindungsgemäß ist es vorgesehen, dass eine Überwachungseinheit (2) mindestens eine Signaleinrichtung zur Abgabe eines nicht sichtbaren Signals aufweist. Ferner ist es vorgesehen, dass die Körperfunktion mittels des nicht sichtbaren Signals kontrolliert wird.

## Beschreibung

Die Erfindung betrifft eine Vorrichtung und ein Verfahren zur Kontrolle einer Körperfunktion, beispielsweise eine Muskcispannung, der Herzrhythmus oder dergleichen. Der Begriff Körperfunktion umfasst aber nicht ausschließlich die vorgenannten Funktionen. Vielmehr sind alle Körperfunktionen umfasst, die messbar sind. Die Erfindung betrifft insbesondere eine Vorrichtung zur Durchführung einer Inkontinenz-Therapie.

Eine oben genannte Vorrichtung ist aus der EP 0 963 217 B1 bekannt, welche eine Vorrichtung zur Inkontinenz-Therapie mittels Elektrostimulation beschreibt. Insbesondere betrifft diese Druckschrift einen Tampon, bestehend aus einem hautverträglichen, elastischen Material, das in angefeuchtetem Zustand entgegen einer Rückstellkraft komprimierbar ist. In das Material des Tampons ist eine unisolierte, elektrisch leitende Elektrode eingeschlossen, die mit einem isolierten, aus dem Material des Tampons heraustretenden Auschlusskabel verbunden ist. Ferner sind bei der bekannten Vorrichtung ein Gerät zur Erzeugung eines Stimulationsstroms (nachfolgend auch Gerät genannt) sowie eine flächige Elektrode vorgesehen, die über ein Elektrodenkabel mit dem einen Pol eines Stromausgangs des Gerätes verbunden ist, wobei der Tampon über das Anschlusskabel mit dem zweiten Pol des Stromausgangs des Gerätes verbunden ist.

Die in das Material des Tampons eingelassene elektrisch leitende Elektrode ermöglicht es, den Tampon als Vaginalelektrode zur Elektrostimulation zu verwenden. Die Elektrostimulationstherapie des Uretharlbereiches dient insbesondere der Kräftigung einer erschlafften Beckenbodenmuskulatur oder des Schließmuskels der Blase, Dabei wird die flächige Elektrode abdominal aufgebracht und der Tampon als stabförmige Elektrode vaginal eingeführt. Der Stromtransport von der Elektrode erfolgt hier innerhalb des angefeuchteten Materials durch Ionentransport innerhalb der Flüssigkeit. Eine ausreichemie Leitfähigkeit des in die Vagina eingeschobenen Tampons während einer großen Zeitdauer ist gewährleistet, da der Tampon aufgrund des durch Körperflüssigkeiten befeuchteten Umfeldes nicht austrocknen kann.

Das Gerät umfasst bei der bekannten Vorrichtung ein Aufnahmefach für eine Batterie, Drehregler zum Regeln des Stimulationsstroms sowie eine digitale Anzeige. Mittels der digitalen Anzeige ist es möglich, die Erzeugung des Stimulationsstroms und somit den Verlauf einer Inkontinenz-Therapie zu überwachen. In der Praxis wird das Gerät außerhalb am Körper sichtbar getragen, damit der Verlauf der Inkontinenz-Therapie mittels der digitalen Anzeige überwacht werden kann.

Von Nachteil ist jedoch, dass das Gerät stets sichtbar getragen werden muss. Somit ist die Verwendung der bekannten Vorrichtung insbesondere für Dritte sichtbar. Da die Verwendung der bekannten Vorrichtung jedoch vielen Anwenderinnen (Patientinnen) unangenehm ist, ziehen sich in der Regel Patientinnen, die eine Inkontinenz-Therapie durchführen müssen, zurück und wenden die Inkontinenz-Therapie nicht bei Anwesenheit Dritter (also in der Öffentlichkeit) an. Eine derartige Therapie sollte allerdings in genauen Zeitabständen und demnach zu gewissen Zeitpunkten angewandt werden, wobei es im Bereich des Wahrscheinlichen liegt, dass eine Patientin sich genau zu einem solchen Zeitpunkt in der Öffentlichkeit aufhält. In diesem Fall würden viele Patientinnen die bekannte Vorrichtung sicherlich nicht verwenden. Dies gefährdet unter Umständen den Erfolg der Inkontinenz-Therapie.

Ferner ist aus dem Stand der Technik das so genannte Biofeedback-Verfahren bekannt. Dieses ist ein Trainingsverfahren, durch das Menschen willentliche Kontrolle über Körperfunktionen erhalten können. Bei dem Biofeedback-Verfahren wird mittels eines Sensors oder mehrerer Sensoren mindestens eine Körperfunktion gemessen. Durch Rückmeldung in Form eines Signals wird diese Körperfunktion einem Menschen "mitgeteilt", Es ist auch bereits bekannt, mittels des Biofeedback- Verfahrens eine Inkontinenz-Therapie durchzuführen. Hierzu wird die Muskelspannung des Beckenbodens bzw. des Schließmuskels der Blase einer Patientin mittels einer Vaginalelektrode oder einer Rektalelektrode unter Verwendung der Elektromyographie gemessen und der Patientin über einen Bildschirm angezeigt, also "zurückgemcidet". Das Training besteht aus einer Sequenz von Muskelanspannungs" und Muskelentspannungsphasen, wobei die Patientin über die Phasen eine entsprechende Meldung auf dem Bildschirm angezeigt erhält.

Das bekannte Verfahren bzw. die bekannte Vorrichtung basierend auf dem Biofeedback- Verfahren weist jedoch ebenfalls den Nachteil auf, dass es wiederum nicht diskret anwendbar ist. Aufgrund des Bildschirms ist die Anwendung des Verfahrens bzw. der Vorrichtung für jeden Dritten ersichtlich, so dass dieselben Nachteile erzielt werden, die bereits hinsichtlich der weiter oben beschriebenen bekannten Vorrichtung hinsichtlich der Elektrostimulation erzielt werden.

Der Erfindung liegt daher die Aufgabe zugrunde, eine Vorrichtung und ein Verfahren zur Kontrolle einer Körperfunktion, insbesondere zur Durchrührung einer Inkontinenz-Therapie zu schaffen, die zu jedem Zeitpunkt diskret verwendbar sind.

Erfindungsgemäß wird diese Aufgabe mit einer Vorrichtung mit den Merkmalen des Anspruchs 1 gelöst. Ein erfindungsgemäßes Verfahren ist durch die Merkmale des Anspruchs 23 charakterisiert. Weitere Ausgestaltungen und Merkmale der Erfindung ergeben sich aus den weiteren Ansprüchen, aus der nachfolgenden Beschreibung und/oder aus den Figuren.

Die erfindungsgemäße Vorrichtung zur Kontrolle einer Körperfunktion ist insbesondere als Vorrichtung zur Durchführung einer Inkontinenz-Therapie ausgebildet, Die erfindungsgemäße Vorrichtung weist mindestens eine Überwachungseinheit zur Kontrolle der Körperfunktion auf. Diese Überwachungseinheit dient demnach insbesondere zur Überwachung des Verlaufs der Inkontinenz-Therapie. Die Überwachungseinheit ist mit mindestens einer Signaleinrichtung zur Abgabe eines nicht sichtbaren Signals versehen.

Die Ausgestaltung und Zusammensetzung der erfindungs gemäßen Vorrichtung weist gegenüber dem Stand der Technik den Vorteil auf, dass die Vorrichtung direkt am Körper einer Patientin getragen werden kann, ohne dass diese sichtbar getragen werden muss. Denn durch das nicht sichtbare Signal, welches die Patientin wahrnimmt, werden der Verlauf der Körperfunktion (beispielsweise eine Muskelspannung) und somit auch der Verlauf einer Therapie (beispielsweise eine Inkontinenz-Therapie) überwacht. Demnach ist es ohne weiteres möglich, die Vorrichtung auch unter einem Kleidungsstück zu tragen. Von Vorteil ist demnach, dass das Tragen der Vorrichtung für Dritte nicht sichtbar ist. Somit ist es möglich, insbesondere eine Inkontinenz-Therapie durchzuführen, ohne dass Dritte hiervon etwas mitbekommen. Einer Patientin wird es somit ermöglicht, die Inkontinenz-Therapie jederzeit durchzuführen, ohne dass die Benutzung der Vorrichtung der Patientin unangenehm sein muss.

Die erfindungs gemäße Vorrichtung ist zur Anwendung mehrerer Verfahren geeignet, insbesondere als Vorrichtung zur Inkontinenz-Therapie mittels Elektrostimulation und als Vorrichtung zur Anwendung eines Biofeedback-Verfahrens als Inkontinenz-Therapie. Insbesondere das letztere ist besonders von Vorteil. Das nicht sichtbare Signal, welches in diesem Falle als "Rückmeldesignal" hinsichtlich der Körperfunktion ausgebildet ist, ist von Dritten nicht ungewollt wahrnehmbar. Dabei ist gemäß der Erfindung vorzugsweise vorgesehen, dass die Körperfunktion, beispielsweise eine Muskelanspannung, gemessen wird (beispielsweise mittels der Elektromyographie) und bei Überschreiten eines vorgegebenen Schwellenwertes das nicht sichtbare Signal als Rückmeldung für das Erreichen einer bestimmten Muskelanspannung abgegeben wird. Die Patientin erhält hierdurch eine deutliche Erkenntnis über den Verlauf der Anwendung. Vorzugsweise ist es auch vorgesehen, dass bei einem Überschreiten eines ersten Schwellenwertes automatisch oder manuell ein zweiter Schwellenwert eingestellt wird, der erreicht werden muss, damit es wieder zu einer Rückmeldung kommt. Dies ist insbesondere in der Inkontinenz-Therapie von Vorteil, da eine kontinuierliche Fortführung der Therapie (Muskeltraining) und eine Steigerung des Trainingsziels (eine gewisse Muskelspannung) gewährleistet sind.

Die Signaleinrichtung der erfindungsgemäßen Vorrichtung gibt vorzugsweise ein nicht sichtbares Signal in Form eines hörbaren Signals ab, dessen Lautstärke einstellbar ist, um sicherzustellen, dass dieses hörbare Signal für Dritte nicht hörbar ist. Alternativ oder zusätzlich hierzu ist vorgesehen, dass die Signaleinrichtung zur Abgabe eines fühlbaren Signals ausgebildet ist. Die Verwendung des fühlbaren Signals ist besonders von Vorteil, da bei Anordnung der Überwachungseinheit direkt am Körper einer Patientin der Verlauf insbesondere einer Inkontinenz-Therapie völlig diskret Überwachbar ist. Die erfindungsgemäße Vorrichtung ist somit völlig diskret auch in der Öffentlichkeit tragbar.

Bei einer Ausführungsform der Erfindung ist die Signaleinrichtung zur Abgabe eines Stimulationsstroms ausgebildet. Dieser wird vorzugsweise als Rückmeidesignal in einem Biofeedback-Verfahren verwendet.

Bei einer weiteren Ausgestaltung der Erfindung ist die Signaleinrichtung mit mindestens einer Einheit zur Erzeugung einer Bewegung versehen. Diese Einheit zur Erzeugung einer Bewegung ist insbesondere zur Abgabe des oben genannten fühlbaren Signals ausgebildet. Bei einer vorteilhaften Ausgestaltung ist die Einheit zur Erzeugung einer Bewegung als Motor, beispielsweise ein Elektromotor, ausgebildet. Des Weiteren ist bei einer weiteren Ausführungsform der Erfindung vorgesehen, dass die Einheit zur Erzeugung einer Bewegung eine Vibrationsbewegung erzeugt. Diese Vibrationsbewegung ist als fühlbares Signal besonders gut geeignet und wird von einer Patientin ohne weiteres sofort wahrgenommen.

Bei einer weiteren Ausgestaltung der Erfindung ist die Überwachungseinheit auch zur Steuerung und/oder Regelung einer Inkontinez-Therapie ausgebildet. Durch entsprechende Einstelltmöiglichkeiten, beispielsweise Regler, ist es möglich, die Erzeugung eines Stimulationsstroms (auch Elektrostimulationsstrom genannt) zu veranlassen und den Stimulationsstrom zu steuern. Auf diese Weise ist es möglich, die Inkontinenz-Therapie zu beeinflussen.

Bei einer weiteren Ausführungsform der Erfindung ist die Überwachungseinheit mit einer Therapieeinheit verbunden. Die Verbindung zwischen der Überwachungseinheit und der Therapieeinheit erfolgt dabei bei einer bevorzugten Ausführungsform drahtlos, beispielsweise über ein so genanntes Wireless LAN oder über eine BlueTooth-Verbindung. Alternativ oder zusätzlich hierzu kann bei einer weiteren Ausführungsform die Verbindung über mindestens eine elektrische Zuführung zwischen der Überwachungseinheit und der Therapieeinheit erfolgen. Vorzugsweise ist es vorgesehen, dass die Therapieeinheit ein hautverträgliches, elastisches Material umfasst, das in angefeuchtetem Zustand entgegen einer Rückstellkraft komprimierbar ist. Besonders bevorzugt ist vorgesehen, dass die Therapieeinheit als Tampon ausgebildet ist.

Bei einer weiteren bevorzugten Ausführungsform der Erfindung ist in der Therapieeinheit mindestens eine unisolierte, elektrisch leitende Elektrode angeordnet, die mit einem isolierten, aus der Therapieeinheit, heraustretenden Anschlusskabel verbunden ist. Vorzugsweise ist vorgesehen, dass in der Therapieeinheit eine Mehrzahl unisolierter, elektrisch leitender Elektroden angeordnet sind, die jeweils mit einem isolierten, aus der Therapieeinheit heraustretenden Anschlusskabel verbunden sind. Ferner ist es möglich, mittels der Elektrode bzw. den Elektroden ein Elektromyogramm zur Bestimmung einer Körperfunktion aufzunehmen.

Bei einer weiteren Ausgestaltung der Erfindung weist das Material der Therapieeinheit eine Rückstellkraft auf, die, bei in eine Vagina eingeschobener Therapieeinheit einen Druck auf die Urethra ausübt, der ausreichend groß zur Vermeidung unwillkürlichen Harnverlustes ist.

Bei einer weiteren Ausgestaltung der Erfindung ist die Überwachungseinheit flächig ausgebildet. Hierunter wird verstanden, dass die Überwachungseinheit möglichst flach mit einer großen Fläche ausgestaltet ist, die am Körper einer Patientin angeordnet werden kann. Die flächige und flache Ausbildung gewährleistet, dass die Überwachungseinheit diskret am Körper getragen werden kann.

Die möglichst flache Ausbildung wird insbesondere dadurch erreicht, dass vorzugsweise die Überwachungseinheit eine erste Längenausdehnung entlang einer ersten Achse und eine zweite Längenausdehnung entlang einer zweiten Achse aufweist, wobei die erste Längenausdehnung größer als die zweite Längenausdehnung ausgebildet ist und wobei die erste Achse zur zweiten Achse senkrecht ausgerichtet ist. Ferner ist vorzugsweise vorgesehen, dass die Überwachungseinheit eine dritte Längenausdehnung entlang einer dritten Achse aufweist, wobei die dritte Achse sowohl senkrecht zur ersten Achse als auch senkrecht zur zweiten Achse ausgerichtet ist und wobei die zweite Längenausdehnung größer als die dritte Längenausdehnung ausgebildet ist.

Bei einer bevorzugten Ausführungsform der Erfindung weist die Überwachungseinheit eine Spannungsversorgungseinheit auf. Vorzugsweise ist die Spannungsversorgungseinheit wiederaufladbar ausgebildet. Diese Ausführungsform gewährleistet, dass die erfindungsgemäße Vorrichtung unabhängig von einer externen Spannungsversorgung einsetzbar ist. Insbesondere durch die Wiederaufladbarkeit der Spannungsversorgungseinheit ist die erfindungsgemäße Vorrichtung wiederverwendbar.

Eine weitere Ausgestaltung der Erfindung sieht vor, dass an der Überwachungseinheit mindestens eine Elektrode angeordnet ist. Besondere Ausführungsformen der Erfindung sehen es vor, dass bis zu 4 Elektroden an der Überwachungseinheit angeordnet sind. Vorzugsweise ist vorgesehen, dass die Elektrode in die Überwachungseinheit integriert ist. Diese Elektrode ist mit einer in der Überwachungseinheit aufgenommenen Steuerungseinheit verbunden, während die Therapieeinheit über ein Anschlusskabel mit der Steuerungseinheit verbunden ist. Mittels der Steuerungseinheit und der Elektroden ist ein Elektrostimulationsstrom erzeugbar. Die vorgenannte Ausführungsform ermöglicht eine möglichst kleine und flache Ausbildung der Überwachungseinheit, so dass die erfindungsgemäße Vorrichtung diskret am Körper getragen werden kann.

Die Erfindung betrifft auch ein Verfahren zur Kontrolle einer Körperfunktion, bei dem die Körperfunktion mittels eines nicht sichtbaren Signals kontrolliert wird. Vorzugsweise ist das nicht sichtbare Signal ein hörbares und/oder ein fühlbares Signal. Beispielsweise ist es vorgesehen, dass als nicht sichtbares Signal eine Vibrationsbewegung und/oder ein Stimulationsstrom verwendet wird/werden. Bei einer weiteren Ausführungsform des erfindungsgemäßen Verfahrens wird das nicht sichtbare Signal bei Überschreiten eines Schwellenwertes der Körperfunktion abgegeben. Der Schwellenwert ist vorzugsweise manuell oder automatisch einstellbar.

Bei einer weiteren Ausführungsform des erfindungsgemäßen Verfahrens werden Faktoren der Körperfunktion ermittelt, wobei die Faktoren der Körperfunktion Faktoren des nicht sichtbaren Signals derart beeinflussen, dass die Faktoren der Körperfunktion zu den Faktoren des nicht sichtbaren Signals in Beziehung stehen. Beispielsweise wird zur Verbesserung des Therapieeffekts die Muskelanspannung mittels eines Elektromyogramms bestimmt und diese hinsichtlich besonderer Faktoren ausgewertet, welche die Qualität der erreichten Muskelanspannung wiedergeben, wobei eine bestimmte Qualität erreicht werden soll, um den Therapieeffekt zu erhöhen. Beispielsweise werden die Dauer und die Intensität der Muskelanspannung als Faktoren ausgewertet. Das Ergebnis der Auswertung dieser Faktoren und somit auch die Qualität der Muskelanspannung werden dann in ein nicht sichtbares Signal, beispielsweise in Form von Vibrationsbewegungen, umgewandelt. Insbesondere ist es vorgesehen, dass die Dauer und/oder die Intensität der Muskelanspannung zu der Dauer und/oder der Intensität des nicht sichtbaren Signals in direkter Beziehung stehen, so dass eine Patientin eine direkte Rückmeldung über die Qualität der erzielten Muskelanspannung erhält. Hierbei ist es insbesondere vorgesehen, dass die Dauer des nicht sichtbaren Signals einer Patientin auch signalisiert, in dieser Zeit keine Muskeln anzuspannen. Die Dauer des nicht sichtbaren Signals bestimmt somit eine Entspannungsphase, welche für die Therapie ebenso wichtig ist, wie die Muskel anspannung.

Die Erfindung wird nachfolgend nun anhand eines Ausführungsbeispiels mittels Figuren näher beschrieben. Es zeigen
- Fig. 1: eine schematische Darstellung einer erfindungsgemäßen Vorrichtung, die in eine Vagina eingeführt ist;
- Fig. 2: eine vergrößerte schematische Darstellung der Vorrichtung gemäß Figur 1;
- Fig. 3: eine schematische Darstellung einer Therapieeinheit in Form eines Tampons;
- Fig. 4: eine schematische Darstellung insbesondere einer Überwachungseinheit;
- Fig. 5: eine schematische Darstellung in den Innenraum der Überwachungseinheit nach Fig. 4; sowie
- Fig. 6a,b: Draufsichten auf die Überwachungseinheit nach Fig. 4.

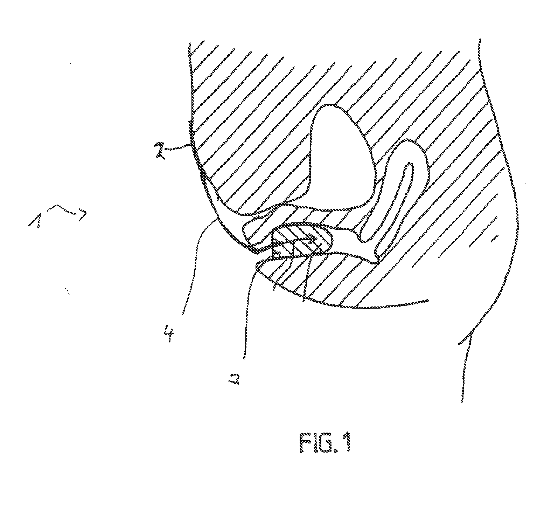

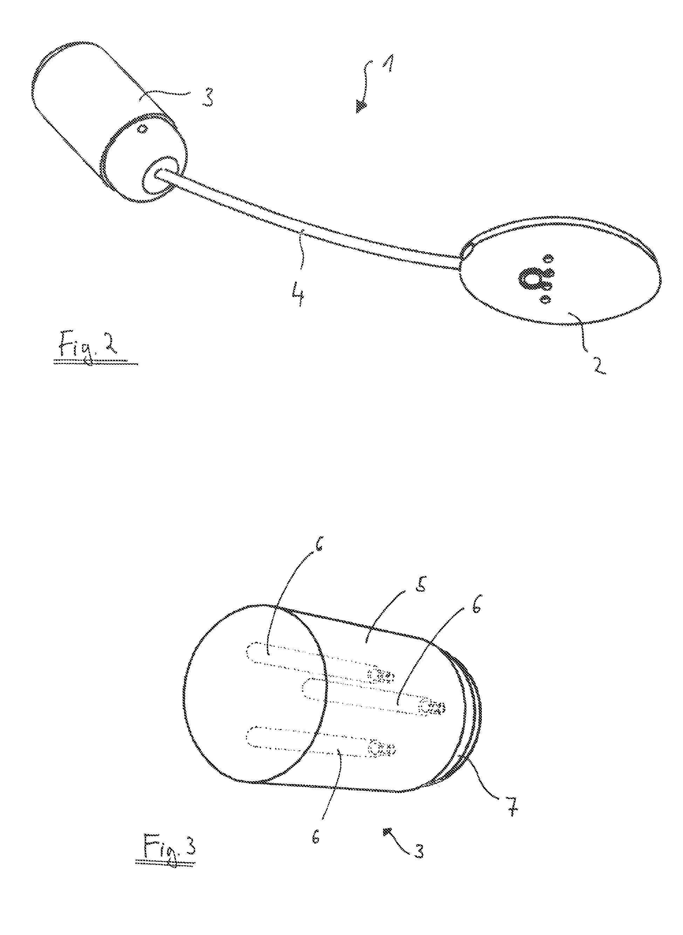

Figur 1 zeigt eine schematische Darstellung einer Vorrichtung 1 zur Therapie der Harninkontinenz von Frauen, welche an einem Körper einer Patientin getragen wird. Die Vorrichtung 1 weist eine flächig ausgebildete Überwachungseinheit 2 auf, welche abdominal am Körper der Patientin angeordnet ist. Ferner ist die Vorrichtung 1 mit einer Therapieeinheit in Form eines Tampons 3 versehen, die in die Vagina der Patientin eingeführt ist. Die Überwachungseinheit 2 und der Tampon 3 sind mittels einer Zuleitung 4 miteinander elektrisch verbunden. Figur 2 zeigt die Vorrichtung 1 in einer weiteren schematischen Darstellung.

Der Tampon 3 ist in Figur 3 näher dargestellt. Er weist im wesentlichen ein Tamponmaterial 5 auf, das aus einem schaumartigen PVA-Material (Polyvenylaikohol) hergestellt wurde. Das Tamponmaterial 5 ist an einer Basis 7 angeordnet, die als Kunstsioff-Vollspritzteil ausgebildet ist. In dem Tamponmaterial 5 sind drei stabförmige Kontakte 6 angeordnet, die als Elektroden für eine Elektrostimulation oder als Messelektroden für eine Elektromyographie vorgesehen sind. Die Erfindung ist allerdings nicht auf die Verwendung von drei Kontakten (Elektroden) 6 eingeschränkt. Vielmehr kann auch eine einzelne Elektrode oder können mehr als drei Elektroden verwendet werden. Die Kontakte 6 sind über eine Anschlussverbindung, die in der Zuleitung 4 angeordnet ist, mit der Überwachungseinheit 2 verbunden.

Das dargestellte Ausführungsbeispiel der erfindungsgemäßen Vorrichtung 1 kann auf zwei Arten betrieben werden, nämlich zum einen als Vorrichtung zur Inkontinenz-Therapie mittels Elektrostimulation und zum anderen als Vorrichtung zur Anwendung eines Biofeedback -Verfahrens bei einer Inkontinenz- Therapie, Die Überwachungseinheit 2 bei dem dargestellten Ausführungsbeispiel weist Bauteile zur Durchführung beider Verfahren auf. Alternativ hierzu ist vorgesehen, dass die erfindungsgemäße Vorrichtung 1 nur Bauteile zur Durchführung eines der genannten Verfahren aufweist.

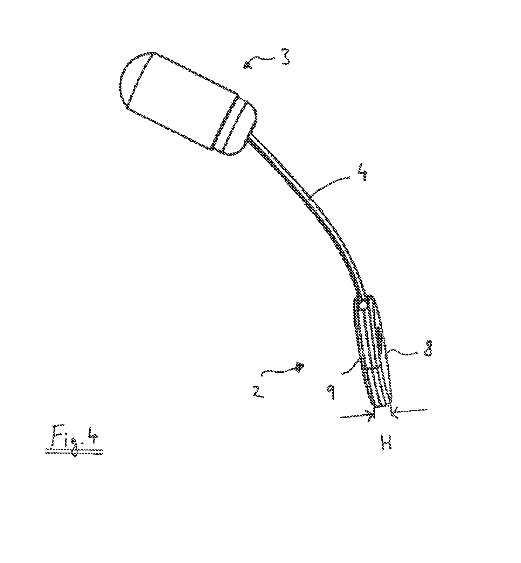

Weitere Einzelheiten der Überwachungseinheit 2 sind in Figur 4 dargestellt. Die Überwachungseinheit 2 dient zum einen der Steuerung und zum anderen der Überwachung der Eiektrostimuiation, wenn das Elektrostimulationsverfahren angewandt wird. Die Überwachungseinheit 2 ist aus einem Kunststoffgehäuse gebildet, das eine Oberseite 8 und eine Unterseite 9 aufweist. Die Oberseite 8 und die Unterseite 9 sind mit einem leitfähigen Kunststoff ummantelt.

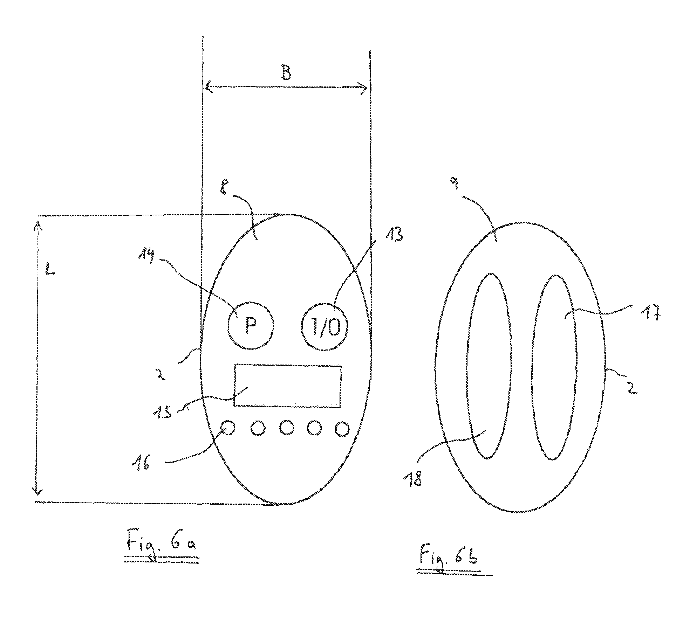

Noch weitere Einzelheiten der Überwachungseinheit 2 sind aus den Figuren 6a und 6b ersichtlich. Aufgrund der Ummantelung mit dem leitfähigen Kunststoff ist es möglich, an der Unterseite 9 zwei Elektroden 17 und 18 anzuordnen, welche parallel zueinander ausgerichtet sind. Alternativ zur Anordnung von zwei Elektroden sehen weitere Ausführungsbeispiele vor, nur eine einzelne oder sogar mehr als zwei Elektroden vorzusehen. Auf der Oberseite 8 ist ein Bedienfeld angeordnet, welches einen Schalter 13 zum Ein- oder Ausschalten der Überwachungseinheit 2 umfasst. Ferner ist ein Regler 14 vorgesehen, mit dessen Hilfe ein Schwellenwert der Muskelspannung einstellbar ist Darüber hinaus weist das Bedienfeld eine Anzeige 15 sowie Kontroll-LEDs 16 auf.

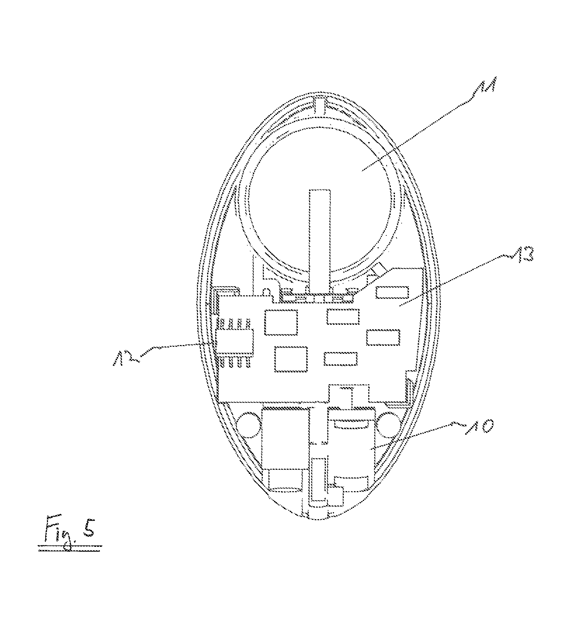

Innerhalb des Kunststoffgehäuses der Überwachungseinheit 2 sind gemäß der Figur 5 eine Spannungsversorgungseinheit in Form einer wiederaufladbaren Batterie 11 sowie eine Elektronikeinheit 12 in Form eines Prozessors angeordnet. Die Elektronikeinheit 12 ist an einer Leiterplatte 13 angeordnet und ermöglicht es, einen Stimulatiortsstrom zu erzeugen. Die Eicktronikeinheit 12 wird dabei mittels des am Kunststoffgehäuse angeordneten Bedienfeldes bedient.

Ferner ist an der Überwachungseinheit 2 ein Motor 10 angeordnet, der ebenfalls mit der Elektronikeinheit 12 verbunden ist. Der Motor 10 dient der Erzeugung einer Vibrationsbewegung. Dabei wird die Vibrationsbewegung in Abhängigkeit des Verlaufs der Elektrostimulation gesteuert. Somit ist es für eine Patientin möglich, den Verlauf der Elektrostimulation und somit der Inkontinenz-Therapie anhand der Vibrationsbewegungen (beispielsweise anhand deren Stärke und Geschwindigkeit) nachzuvollzichen.

Wie oben bereits erwähnt, dient alternativ die Vorrichtung 1 der Ausführung des Biofeedback-Verfahrens bei einer Inkontinenz-Therapie. Hierbei wird mittels der Kontakte 6 ein Blektromyogramm erstellt, mit der die Muskelanspannung der Patientin bestimmt wird. Sobald die Muskel anspannung einen bestimmten vorgegebenen Wert überschreitet, wird ein nicht sichtbares Signal in Form einer Vibrationsbewegung als "Rückmeldesignal" ausgegeben. Alternativ oder zusätzlich hierzu kann auch ein Stimulationsstrom abgegeben werden, Zur Verbesserung des Therapieeffektes wird bei Erreichen des vorgegebenen Wertes der Schwellenwert durch die Elektronikeinheit 12 auf einen höheren Wert neu eingestellt.

Bei einer weiteren Ausführungsform des erfindungsgemäßen Verfahrens, welches bei der Vorrichtung 1 eingesetzt wird, wird zur Verbesserung des Therapieeffekts mittels eines Elektromyogramms die Muskelanspannung bestimmt und diese hinsichtlich besonderer Faktoren ausgewertet, welche die Qualität der erreichten Muskelanspannung wiedergeben, wobei eine bestimmte Qualität erreicht werden soll, um den Therapieeffekt zu erhöhen. Beispielsweise werden die Dauer und die Intensität der Muskelanspannung als Faktoren ausgewertet. Das Ergebnis der Auswertung dieser Faktoren und somit auch die Qualität der Muskelanspannung werden dann in ein nicht sichtbares Signal, beispielsweise in Form von Vibrationsbewegungen, umgewandelt. Insbesondere ist vorgesehen, dass die Dauer und/oder die Intensität der Muskelanspannung zu der Dauer und/oder der Intensität des nicht sichtbaren Signals in direkter Beziehung stehen, so dass eine Patientin eine direkte Rückmeldung über die Qualität der erzielten Muskelanspannung erhält. Hierbei ist es insbesondere vorgesehen, dass die Dauer des nicht sichtbaren Signals einer Patientin auch signalisiert, in dieser Zeit keine Muskeln anzuspannen. Die Dauer des nicht sichtbaren Signals bestimmt somit eine Entspannungsphase, welche für die Therapie ebenso wichtig ist, wie die Muskelanspannung.

Das vorheschriebene Ausführungsbeispiel der Überwachungseinheit ist recht klein ausgebildet. Es weist eine Länge L von ca. 65mm und eine Breite B von ca. 35 auf (vg1. Figur 6a). Die Höhe H beträgt ca. 10mm (vg1. Figur 4).

Die erfindungsgemäße Vorrichtung 1 weist den Vorteil auf, dass die Vorrichtung 1 direkt am Körper der Patientin getragen werden kann, wie es beispielsweise in Fig. 1 dargestellt ist. Es ist sofort ersichtlich, dass die Vorrichtung 1 nicht sichtbar getragen werden muss. Sie ist derart ausgestaltet, dass sie mittels eines Kleidungsstücks verdeckt getragen werden kann. Durch das nicht sichtbare Signal in Form der Vibrationsbewegung oder des Stimulationsstroms, welches die Patientin wahrnimmt, wird der Verlauf der Inkontinenz-Therapie überwacht. Von Vorteil ist auch, dass das Tragen der Vorrichtung 1 für Dritte nicht sichtbar ist. Somit ist es möglich, die Inkontinenz-Therapie durchzuführen, ohne dass Dritte hiervon etwas mitbekommen.

Alternativ oder auch zusätzlich zu der Verwendung der Vibrationsbewegung ist es auch vorgesehen, eine Abgabe eines hörbaren Signals zu verwenden, wobei die Lautstärke dieses hörbaren Signals vorzugsweise einstellbar ist.

### Bezugszeichenliste

- 1: Vorrichtung
- 2: Überwachungseinheit
- 3: Tampon
- 4: Zuleitung
- 5: Tamponmaterial
- 6: Kontakt
- 7: Basis
- 8: Oberseite
- 9: Unterseite
- 10: Motor
- 11: Batterie
- 12: Elektronikeinheit
- 13: Ein- und Ausschalter
- 14: Schwellenschaher
- 15: Anzeige
- 16: LEDs
- 17: Elektrode
- 18: Elektrode

## Patentansprüche

1. Vorrichtung (1) zur Kontrolle einer Körperfunktion, wobei die Vorrichtung (1) mindestens eine Überwachungseinheit (2) zur Überwachung der Körperfunktion aufweist,
**dadurch gekennzeichnet,**
**dass** die Überwachungseinheit (2) mindestens eine Signaleinrichtung (3, 6, 10, 12) zur Abgabe eines nicht sichtbaren Signals aufweist.

2. Vorrichtung (1) nach Anspruch 1, **dadurch gekennzeichnet, dass** die Signaleinrichtung zur Abgabe eines hörbaren Signals ausgebildet ist.

3. Vorrichtung (1) nach Anspruch 1 oder 2, **dadurch gekennzeichnet, dass** die Signaleinrichtung (3, 6, 10, 12) zur Abgabe eines fühlbaren Signals ausgebildet ist.

4. Vorrichtung (1) nach Anspruch 3, **dadurch gekennzeichnet, dass** die Signaleinrichtung (3, 6, 12) zur Abgabe eines Stimulationsstroms ausgebildet ist.

5. Vorrichtung (1) nach einem der vorangehenden Ansprüche, **dadurch gekennzeichnet, dass** die Signaleinrichtung (1) mindestens eine Einheit (10) zur Erzeugung einer Bewegung aufweist.

6. Vorrichtung (1) nach Anspruch 5, **dadurch gekennzeichnet, dass** die Einheit zur Erzeugung einer Bewegung als Motor (10) ausgebildet ist.

7. Vorrichtung (1) nach Anspruch 5 oder 6, **dadurch gekennzeichnet, dass** die Einheit (10) zur Erzeugung einer Bewegung zur Abgabe einer Vibrationsbewegung ausgebildet ist.

8. Vorrichtung (1) nach einem der vorangehenden Ansprüche, **dadurch gekennzeichnet, dass** die Überwachungseinheit (2) zur Steuerung einer Inkontinenz-Therapie ausgebildet ist.

9. Vorrichtung (1) nach einem der vorangehenden Ansprüche, **dadurch gekennzeichnet, dass** die Überwachungseinheit (2) mit einer Therapieeinheit (3) verbunden ist.

10. Vorrichtung (1) nach Anspruch 9, **dadurch gekennzeichnet, dass** die Überwachungseinheit (2) mit der Therapieeinheit (3) drahtlos und/oder über mindestens eine elektrische Zuführung (4) verbunden ist,

11. Vorrichtung (1) nach Anspruch 9 oder 10, **dadurch gekennzeichnet, dass** die Therapieeinheit (3) ein hautverträgliches, elastisches Material (5) aufweist, das in angefeuchtetem Zustand entgegen einer Rückstellkraft komprimierbar ist.

12. Vorrichtung (1) nach einem der Ansprüche 9 bis 11, **dadurch gekennzeichnet, dass** die Therapieeinheit (3) als Tampon ausgebildet ist.

13. Vorrichtung (1) nach einem der Ansprüche 9 bis 12, **dadurch gekennzeichnet, dass** in der Therapieeinheit (3) mindestens eine unisolierte, elektrisch leitende Elektrode (6) angeordnet ist, die mit einem isolierten, aus der Therapieeinheit (3) heraustretendem Anschlusskabel verbunden ist.

14. Vorrichtung (1) nach Anspruch 13, **dadurch gekennzeichnet, dass** in der Therapieeinheit (3) eine Mehrzahl unisolierter, elektrisch leitender Elektroden (6) angeordnet ist, die jeweils mit einem isolierten, aus der Therapieeinheit (3) heraustretenden Anschlusskabel verbunden sind,

15. Vorrichtung (1) nach einem der Ansprüche 11 bis 14, **dadurch gekennzeichnet, dass** das Material (5) der Therapieeinheit (3) eine Rückstellkraft aufweist, die bei in eine Vagina eingeschobener Therapieeinheit (3) einen Druck auf die Urethra ausübt, der ausreichend groß zur Vermeidung unwillkürlichen Harnverlustes ist.

16. Vorrichtung (1) nach einem der vorangehenden Ansprüche, **dadurch gekennzeichnet, dass** die Überwachungseinheit (2) flächig ausgebildet ist.

17. Vorrichtung (1) nach einem der vorangehenden Ansprüche, **dadurch gekennzeichnet, dass** die Überwachungseinheit (2) eine erste Längenausdehnung (L) entlang einer ersten Achse (L) und eine zweite Längenausdehnung (B) entlang einer zweiten Achse (B) aufweist, wobei die erste Längenausdehnung (L) größer als die zweite Längenausdehnung (B) ausgebildet ist und wobei die erste Achse (L) zur zweiten Achse (B) senkrecht ausgerichtet ist.

18. Vorrichtung (1) nach Anspruch 17, **dadurch gekennzeichnet, dass** die Überwachungseinheit (2) eine dritte Längenausdehnung (H) entlang einer dritten Achse (H) aufweist, wobei die dritte Achse (H) sowohl senkrecht zur ersten Achse (L) als auch senkrecht zur zweiten Achse (B) ausgerichtet ist und wobei die zweite Längenausdehnung (B) größer als die dritte Längenausdehnung (H) ausgebildet ist.

19. Vorrichtung (1) nach einem der vorangehenden Ansprüche, **dadurch gekennzeichnet, dass** die Überwachungseinheit (2) eine Spannungsversorgnngseinheit (11) aufweist.

20. Vorrichtung (1) nach Anspruch 19, **dadurch gekennzeichnet, dass** die Spannungsversorgungseinheit (11) wiederaufladbar ausgebildet ist.

21. Vorrichtung (1) nach einem der vorangehenden Ansprüche, **dadurch gekennzeichnet, dass** an der Überwachungseinheit (2) mindestens eine Elektrode (17, 18) angeordnet ist.

22. Vorrichtung (1) nach Anspruch 21, **dadurch gekennzeichnet, dass** die Elektrode (17, 18) in die Überwachungseinheit (2) integriert ist.

23. Verfahren zur Kontrolle einer Körperfunktion,
**dadurch gekennzeichnet,**
**dass** die Körperfunktion mittels eines nicht sichtbaren Signals kontrolliert wird.

24. Verfahren nach Anspruch 23, **dadurch gekennzeichnet, dass** als nicht sichtbares Signal ein hörbares Signal und/oder ein fühlbares Signal verwendet wird/werden.

25. Verfahren nach Anspruch 24, **dadurch gekennzeichnet, dass** als nicht sichtbares Signal eine Vibrationsbewegung und/oder ein Stimulationsstrom verwendet wird/werden.

26. Verfahren nach einem der Ansprüche 23 bis 25, **dadurch gekennzeichnet, dass** das nicht sichtbare Signal bei Überschreiten eines Schwellenwertes der Körperfunktion abgegeben wird.

27. Verfahren nach Anspruch 26, **dadurch gekennzeichnet dass** der Schwellenwert manuell oder automatisch einstellbar ist,

28. Verfahren nach einem der Ansprüche 23 bis 27, **dadurch gekennzeichnet, dass** Faktoren der Körperfunktion ermittelt werden, wobei die Faktoren der Körperfunktion Faktoren des nicht sichtbaren Signals derart beeinflussen, dass die Faktoren der Körperfunktion zu den Faktoren des nicht sichtbaren Signals in Beziehung stehen.
